# EUROPEAN PATENT SPECIFICATION

(11) **EP 4 308 991 B1**
(45) Date of publication and mention of the grant of the patent: **12.08.2026**
(21) Application number: 22716176.7
(22) Date of filing: 16.03.2022
(51) Int. Cl.: G02B 21/36, G02B 21/00, A61B 34/00, A61B 90/20

(54) **SURGICAL MICROSCOPE SYSTEM AND CORRESPONDING SYSTEM, METHOD AND COMPUTER PROGRAM**
CHIRURGISCHES MIKROSKOPSYSTEM UND ENTSPRECHENDES SYSTEM, VERFAHREN UND COMPUTERPROGRAMM
SYSTÈME DE MICROSCOPE CHIRURGICAL ET SYSTÈME, PROCÉDÉ ET PROGRAMME INFORMATIQUE CORRESPONDANTS

(30) Priority: 18.03.2021 DE 102021106673
(43) Date of publication of application: 24.01.2024
(73) Proprietor: Leica Instruments (Singapore) Pte. Ltd., 608924 Singapore (SG); Leica Microsystems CMS GmbH, 35578 Wetzlar (DE)
(72) Inventor: TEN HAVE, Peter, 35578 Wetzlar (DE); VIKAL, Siddharth, 35578 Wetzlar (DE); DUNLAP, Edward, 35578 Wetzlar (DE); WIETHOFF, Alexander, 35578 Wetzlar (DE); RENNER, Bastian, 35578 Wetzlar (DE); DITTRICH, Svenja, 35578 Wetzlar (DE); THALHAMMER, Veronika, 35578 Wetzlar (DE)
(74) Representative: 2SPL Patentanwälte PartG mbB
(86) International application number: PCT/EP2022/056888
(87) International publication number: WO 2022/194966

(56) References cited:
- EP-A2- 3 785 661
- WO-A1-2014/089494
- WO-A1-2020/154351
- US-A1- 2010 245 557
- US-A1- 2014 327 757
- BARTCZAK PIOTR ET AL: "Spectral Video in Image-Guided Microsurgical Applications: Integrating Imaging Technology into the Clinical Environment and Ergonomic Considerations", 2018 IEEE 31ST INTERNATIONAL SYMPOSIUM ON COMPUTER-BASED MEDICAL SYSTEMS (CBMS), IEEE, 18 June 2018 (2018-06-18), pages 286 - 291, XP033375685, [retrieved on 20180720], DOI: 10.1109/CBMS.2018.00057

## Description

### Technical field

Examples relate to a surgical microscope system, and to a corresponding system, method and computer program for a surgical microscope system.

### Background

Surgical microscope systems are complex devices that provide a large number of functionalities. These functionalities are often accessible via haptic buttons, such as buttons that are located at handles of the surgical microscope system, or buttons that are arranged on a foot pedal of the surgical microscope system. In some cases, access to the functionality may be provided visually, e.g., via a display and a corresponding touch-based input device.

Surgical microscope systems are often equipped to perform imaging in various imaging modes, such as a reflectance imaging mode and one or more different fluorescence imaging modes. Each of the modes enables a unique view on the surgical site, with each mode being suitable for different surgical procedures or different parts of a surgical procedure.

US 2010/245557 A1 discloses a method for injecting secondary images into viewing fields of surgical microscopes are disclosed. Some exemplary embodiments may provide secondary image injection in a picture-in-picture arrangement, thereby allowing a surgeon to simultaneously view the one or more secondary images and the surgical field through the oculars of the surgical microscope.

US 2014/327757 A1 discloses a microscope rendering digital images of samples, with the capability to digitally control the focus of the microscope system, and the software used to control the operation of the digital microscope system.

EP 3 785 661 A2 discloses a method involving capturing, by a two-dimensional or three-dimensional video camera, current video images and recording the captured video images.

A user interface displays the current video images and the recorded video images and/or the three-dimensional model and enables a user to interact with either or both of them.

WO 2020/154351 A1 discloses a system directing an imaging device to continuously capture visible light and fluorescence illumination from a surgical area. The system generates a visible light image stream based on the captured visible light and a fluorescence image stream based on the captured fluorescence illumination. The system operates in a first display mode by directing a display device to display a first video stream based on a first set of at least one of the visible light image stream and the fluorescence image stream. In response to detecting an event that occurs within the surgical area, the system switches from operating in the first display mode to operating in a second display mode by directing the display device to display a second video stream based on a second set of at least one of the visible light image stream and the fluorescence image stream, the first set being different than the second set.

Biotr Bartczak et al. et al. disclose in "Spectral Video in Image-Guided Microsurgical Applications: Integrating Imaging Technology into the Clinical Environment and Ergonomic Considerations", 2018 IEEE 31ST INTERNATIONAL SYMPOSIUM ON COMPUTER-BASED MEDICAL SYSTEMS (CBMS), IEEE, 18 June 2018 (2018-06-18), pages 286-291, DOI: 10.1109/CBMS.2018.00057, a surgical microscope system which uses real-time spectral imaging in an operation room.

### Summary

Various examples of the present disclosure are based on the finding that surgical microscope systems are often capable of providing a multitude of different views on the surgical site. Apart from different imaging modes being used, image processing may be used to create additional views on the sample, which are suitable for different surgical procedures or different parts of a surgical procedure. Additionally, views that are based on image data of external devices or computer-generated views may be used. However, these different views may remain unused if the surgeon or assistant is unable to instantly access or combine the available views. Various aspects of the present disclosure thus provide a concept for the selection of different views on the surgical site via an intuitive user interface, in which preview representations are used to illustrate the available views to the surgeon or assistant selecting the respective view.

Various aspects of the present disclosure relate to a system for a surgical microscope system. The system comprises one or more processors and one or more storage devices. The system is configured to obtain imaging sensor data from at least one optical imaging sensor of a microscope of the surgical microscope system. The system is configured to generate at least one of two or more video streams based on the imaging sensor data. The system is configured to obtain a sensor signal from a touch interface of a touch screen of the microscope system. The sensor signal represents a touch input obtained via the touch interface. The system is configured to generate a display signal for a display of the touch screen of the microscope system. The display signal comprises, in a configuration display mode, a visual configuration interface, the visual configuration interface being controlled via the touch input obtained via the touch interface, and, in a presentation display mode, a representation of the at least one of the two or more video streams. The visual configuration interface comprises a touch-activated control element for selecting the at least one of the two or more video streams for display in the presentation display mode, the touch-activated control element showing a preview representing the two or more video streams. By providing a visual configuration interface with a touch-activated control element, the representation of the at least one of the two or more video streams in the presentation display mode can be modified via an intuitive touch-based user interface. By providing the visual configuration interface with the preview representing the two or more video stream, the user, such as the surgeon or the assistant, is provided with an overview of the available video streams (i.e. views) being available for selection.

In some examples, the system is configured to generate the visual configuration interface such that two of the two or more video streams are selectable via the touch-activated control element for concurrent display in the presentation display mode. For example, the two video streams may be shown side-by-side or in a picture-in-picture mode, allowing for more information being presented simultaneously.

For example, the system may be configured to generate the visual configuration interface such that two of the two or more video streams are selectable via the touch-activated control element for concurrent display via a picture-by-picture mode and/or picture-in-picture mode in the presentation display mode. In the picture-by-picture mode, the two video streams can be seen with equal prominence. In the picture-in-picture mode, one of the video streams is shown with more prominence in a full-screen representation, and the other video stream is shown in a window overlaid over the full-screen representation, providing an additional glimpse on the information contained in the other video stream.

In some cases, it may be desirable to show one of the video streams as large as possible, e.g., in a full-screen representation (which may be extended with a smaller overlay window in a picture-in-picture mode). For example, the system may be configured to generate the visual configuration interface such that one of the two or more video streams is selectable via the touch-activated control element for a full-screen mode in the presentation display mode.

The system may be configured to generate the visual configuration interface such that the at least one of the two or more video streams is selectable via the touch-activated control element using a drag-and-drop operation. A drag-and-drop operation may provide the user with an intuitive way of arranging the video streams for the representation shown in the presentation display mode.

In some cases, the video streams may not only be generated based on the imaging sensor data of the optical imaging sensor. For example, in addition to the imaging sensor data, image data of an external device or computer-generated image data may be used to generate one or more of the video streams. For example, the system may be further configured to obtain image data from an external device and/or by generating the image data from a data source. The system may be configured to generate the at least one of the two or more video streams further based on the image data from the external device and/or based on the image data generated from a data source. In effect, the two or more video streams may comprise at least one video stream that is generated based on the imaging sensor data and at least one video stream that is generated based on the image data from the external device and/or based on the image data generated from a data source.

For example, one or more of the following image data sources may be used. For example, the image data may comprise at least one of image data of an endoscope, image data of an image-guided surgery system, image data of a neurological monitoring system, image data of a pre-operative plan, and image data for generating a visual overlay.

In some examples, the surgical microscope system is suitable for performing fluorescence imaging in one or more separate fluorescence emission wavelength bands. The system may be configured to generate a separate visual configuration interface for each of the one or more separate fluorescence emission bands. For example, separate visual configuration interfaces may be used to allow for different setups in different fluorescence imaging modes.

Additionally, the surgical microscope system may be suitable for performing reflectance imaging. The system may be configured to generate a separate visual configuration interface for video streams being based on the optical imaging data representing a reflectance image. Again, separate visual configuration interfaces may be used to allow for different setups for the reflectance and fluorescence imaging modes.

In general, the two or more video streams might contain not only one video stream per imaging mode, but multiple video streams, such as a color video stream and a monochrome video stream, a video stream with an enhanced contrast, video streams in which only the fluorescence emissions are shown etc. For example, the surgical microscope system is suitable for performing fluorescence imaging in one or more separate fluorescence emission wavelength bands. The two or more video streams may comprise, for each of the one or more separate fluorescence emission wavelength bands, two or more video streams being based on the imaging sensor data. For example, the two or more video streams may comprise, for each of the one or more separate fluorescence emission wavelength bands, a first video stream that is based on a first processed version of the imaging sensor data showing a pseudo-colored representation of the fluorescence emissions in the fluorescence emission wavelength band overlaid over a reflectance image, and a second video stream that is based on a second processed version of the imaging sensor data showing an isolated representation of the fluorescence emissions in the fluorescence emission wavelength band. For example, the first video stream may provide a view that is similar to what is being shown in a reflectance image, with the features of interest (that are infused with a fluorescent dye) being highlighted, while the second video stream may provide a view where the features of interest are shown in isolation.

In various examples, the visual configuration interface for a fluorescence emission wavelength band shows a preview of the two or more video streams that are based on the imaging sensor data of the fluorescence emission wavelength bands. For example, the preview may be used, by the user, to select one of the versions that is most suitable for the task as hand.

For example, the preview representing the two or more video streams may be provided as static images representing the respective video stream. The static images may be used to convey the purpose of the respective video stream, without taxing the system while generating live preview images. Alternatively, the preview representing the two or more video streams may be provided based on the two or more video streams. This may provide a live preview, at the expense of additional computational complexity for simultaneously generating multiple video streams.

In various examples, the visual configuration interface comprises a preview of the resulting representation of the at least one of the two or more video streams. This may be used, by the user, to evaluate the appearance of the representation shown in the presentation display mode.

In general, the configuration display mode is suitable for controlling the representation of the at least one of the two or more video streams. In the presentation display mode, the representation of the at least one of the two or more video streams, as configured using the visual configuration interface, is shown.

Various aspect of the present disclosure relate to a surgical microscope system comprising the system presented above.

Various aspects of the present disclosure relate to a corresponding method for a surgical microscope system. The method comprises obtaining imaging sensor data from at least one optical imaging sensor of a microscope of the surgical microscope system. The method comprises generating at least one of two or more video streams based on the imaging sensor data. The method comprises obtaining a sensor signal from a touch interface of a touch screen of the microscope system. The sensor signal represents a touch input obtained via the touch interface. The method comprises generating a display signal for a display of the touch screen of the microscope system. The display signal comprises, in a configuration display mode, a visual configuration interface, the visual configuration interface being controlled via the touch input obtained via the touch interface, and, in a presentation display mode, a representation of the at least one of the two or more video streams. The visual configuration interface comprises a touch-activated control element for selecting the at least one of the two or more video streams for display in the presentation display mode, the touch-activated control element showing a preview representing the two or more video streams.

Various aspects of the present disclosure relate to a corresponding computer program with a program code for performing the above method when the computer program is executed on a processor.

### Short description of the Figures

Some examples of apparatuses and/or methods will be described in the following by way of example only, and with reference to the accompanying figures, in which
- Fig. 1a: shows a block diagram of an example of a system for a surgical microscope system;
- Fig. 1b: shows a schematic diagram of a surgical microscope system comprising a system for a surgical microscope system;
- Fig. 1c: shows a schematic diagram of an example of a visual configuration interface;
- Fig. 2: shows a flow chart of an example of a method for a surgical microscope system;
- Fig. 3a: shows a schematic drawing of an example of a user interface for a microscope system;
- Fig. 3b: shows a schematic drawing of an example of a user interface for a microscope system, in which an exemplary positioning of various elements is illustrated;
- Fig. 3c: shows an example of a visual indicator of a user interface;
- Figs. 3d and 3e: shows schematic drawings of an example of a user interface for a microscope system, in which a picture-in-picture mode is shown;
- Figs. 3f: shows a schematic drawing of an example of a user interface for a microscope system, in which a picture-by-picture mode is shown;
- Fig. 3g: shows a schematic drawing of an example of a user interface for a microscope system, in which a placement of a visual indicator is shown;
- Fig. 3h: shows a schematic drawing of an example of a transition between screens of an exemplary user interface; and
- Fig. 4: shows a schematic diagram of a system comprising a microscope and a computer system.

### Detailed Description

Various examples will now be described more fully with reference to the accompanying drawings in which some examples are illustrated. In the figures, the thicknesses of lines, layers and/or regions may be exaggerated for clarity.

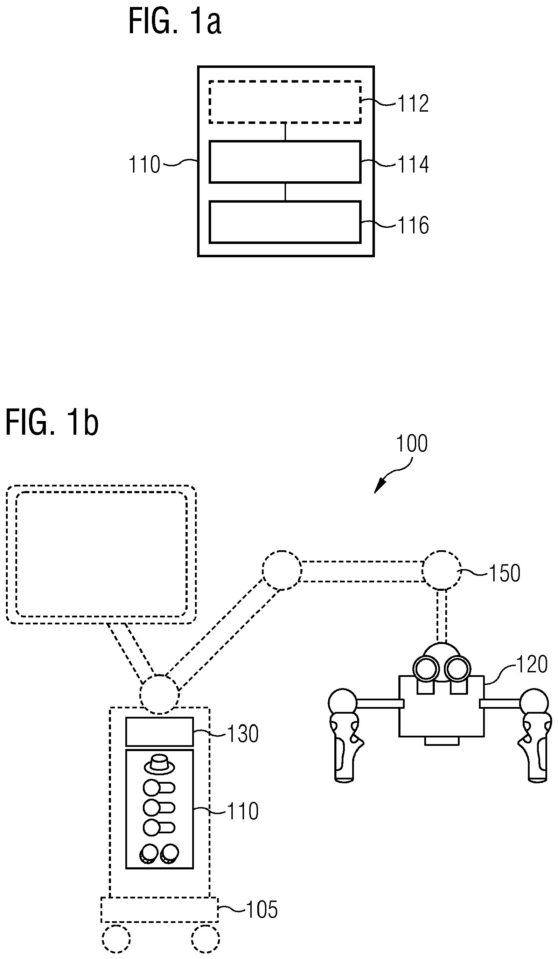

Fig. 1a shows a block diagram of an example of a system 110 for a surgical microscope system 100. The system 110 comprises one or more processors 114 and one or more storage devices 116. Optionally, the system further comprises one or more interfaces 112. The one or more processors 114 are coupled to the one or more storage devices 116 and to the optional one or more interfaces 112. In general, the functionality of the system is provided by the one or more processors, in conjunction with the one or more interfaces (for exchanging information, e.g., with an optical imaging sensor of a microscope) and/or with the one or more storage devices (for storing and/or retrieving information).

The system is configured to obtain (e.g., receive) imaging sensor data from at least one optical imaging sensor of a microscope 120 of the surgical microscope system. The system is configured to generate (e.g., compute) at least one of two or more video streams based on the imaging sensor data. The system is configured to obtain (e.g., receive) a sensor signal from a touch interface of a touch screen 130 of the microscope system. The sensor signal represents a touch input obtained via the touch interface. The system is configured to generate a display signal for a display of the touch screen of the microscope system. The display signal comprises, in a configuration display mode, a visual configuration interface 140. The visual configuration interface is controlled via the touch input obtained via the touch interface. The display signal comprises, in a presentation display mode, a representation of the at least one of the two or more video streams. The visual configuration interface comprises a touch-activated control element 142 for selecting the at least one of the two or more video streams for display in the presentation display mode, the touch-activated control element showing a preview 144 representing the two or more video streams.

Embodiments of the present disclosure relate to a system, method and computer program for a surgical microscope system. In general, a microscope is an optical instrument that is suitable for examining objects that are too small to be examined by the human eye (alone). For example, a microscope may provide an optical magnification of a sample. In modern microscopes, the optical magnification is often provided for a camera or an imaging sensor, such as an optical imaging sensor of the microscope 120 that is shown in Fig. 1b. The microscope 120 may further comprise one or more optical magnification components that are used to magnify a view on the sample, such as an objective (i.e. lens).

There are a variety of different types of microscopes. If the microscope is used in the medical or biological fields, the object being viewed through the microscope may be a sample of organic tissue, e.g., arranged within a petri dish or present in a part of a body of a patient. In the context of the present disclosure, the microscope 120 may be part of a (neuro)surgical microscope system 100, e.g., a microscope to be used during a (neuro)surgical procedure. Such a system is shown in Fig. 1b, for example. Accordingly, an object being viewed through the microscope, and shown in the imaging sensor data, may be a sample of organic tissue of a patient. Although embodiments are described in connection with a microscope, they may also be applied, in a more general manner, to any optical device.

The above system 110 is suitable for use with the surgical microscope system comprising the microscope 120, e.g., as part of the surgical microscope system 100. Fig. 1b shows a block diagram of the surgical microscope system 100 comprising the system 110, the microscope 120 and the touch screen 130. The microscope system shown in Fig. 1b is a surgical microscope system. However, the system 110 may be used with other microscope systems or optical systems as well. The surgical microscope system 100 shown in Fig. 1b comprises a number of optional components, such as a base unit 105 (comprising the system 110) with a (rolling) stand, the touch-screen 130, a (robotic or manual) arm 150 which holds the microscope 120 in place, and which is coupled to the base unit 105 and to the microscope 120, and steering handles 160 that are attached to the microscope 120. For example, the touch screen 130 may be arranged at the base unit 105 of the microscope system. Alternatively, the touch-screen may be arranged at the microscope of the surgical microscope system, e.g., as auxiliary display facing the surgeon or facing an assistant. In the context of this application, the term "surgical microscope system" is used, in order to cover the portions of the system that are not part of the actual microscope (which comprises optical components), but which are used in conjunction with the microscope, such as the touch-screen or an illumination system.

The system is configured to obtain imaging sensor data from the optical imaging sensor of the microscope. For example, the optical imaging sensor may comprise or be an APS (Active Pixel Sensor) - or a CCD (Charge-Coupled-Device)-based imaging sensor. For example, in APS-based imaging sensors, light is recorded at each pixel using a photo-detector and an active amplifier of the pixel. APS-based imaging sensors are often based on CMOS (Complementary Metal-Oxide-Semiconductor) or S-CMOS (Scientific CMOS) technology. In CCD-based imaging sensors, incoming photons are converted into electron charges at a semiconductor-oxide interface, which are subsequently moved between capacitive bins in the imaging sensors by a control circuitry of the imaging sensors to perform the imaging. In various examples, the optical imaging sensor may comprise two or more individual sensors, e.g., two or more sensors for performing stereoscopic imaging, and/or two or more sensors for recording light in different wavelength bands. The system is configured to obtain (i.e. receive or read out) the imaging sensor data from the optical imaging sensor. The imaging sensor data may be obtained by receiving the imaging sensor data from the optical imaging sensor (e.g., via the interface 112), by reading the imaging sensor data out from a memory of the optical imaging sensor (e.g., via the interface 112), or by reading the imaging sensor data from a storage device 116 of the system 110, e.g., after the imaging sensor data has been written to the storage device 116 by the optical imaging sensor or by another system or processor.

The proposed concept is based on the insight, that a preview of the available video streams, and, optionally, a preview of the resulting representation, can provide an intuitive way for a user to configure the representation shown in the presentation display mode. In general, the two or more video streams, or at least a subset of the two or more video streams, are generated by processing the imaging sensor data, in order to generate two or more processed versions of the imaging sensor data. In other words, the two or more video streams may comprise two or more video streams that are based on processed versions of the imaging sensor data. In this context, the term "processed versions" of imaging sensor data indicates, that image processing is being applied on the imaging sensor data to generate the two or more video streams. In various examples, the imaging sensor data comprises raw sensor data, which comprises intensity values separately for different wavelength bands being recorded by the optical imaging sensor. In a simple example, in case a Red-Green-Blue (RGB) sensor is used, the imaging sensor data may comprise intensity values separately for a Red wavelength band, a Green wavelength band and a Blue wavelength band (i.e. for light recorded having a wavelength that is encompassed by the respective R/G/B wavelength bands). Alternatively, different wavelength bands may be used, or a larger number of wavelength bands. While some (or all) of the wavelength bands may be used for reflectance imaging (i.e. the recording of light that is reflected off a sample), some may be used for fluorescence imaging (i.e. the recording is light that is emitted by the sample through fluorescence emissions). In other words, the surgical microscope system may be suitable for performing reflectance imaging. Accordingly, one of the two or more video streams may be based on reflectance imaging. Additionally, the surgical microscope system may be suitable for performing fluorescence imaging in one or more separate fluorescence emission wavelength bands (or two or more separate fluorescence emission bands). In surgical microscope systems, both reflectance imaging and fluorescence imaging may be used at the same time, to provide a reflectance image (also called "white light" image) with a pseudo-color overlay that is based on the fluorescence emissions. One of the video streams may be based on reflectance imaging, overlaid with a fluorescence emission overlay. Another of the video streams may be a video stream, in which the fluorescence emissions are shown in isolation. In other words, the two or more video streams may comprise, for each of the one or more separate fluorescence emission wavelength bands, a first video stream that is based on a first processed version of the imaging sensor data showing a pseudo-colored representation of the fluorescence emissions in the fluorescence emission wavelength band overlaid over a reflectance image, and a second video stream that is based on a second processed version of the imaging sensor data showing an isolated representation of the fluorescence emissions in the fluorescence emission wavelength band. Consequently, the two or more video streams may comprise, for each of the one or more separate fluorescence emission wavelength bands, two or more video streams being generated based on the imaging sensor data. Additionally, the two or more video streams may comprise a video stream that is based on reflectance imaging, without a pseudo-color overlay. To sum up, the same imaging sensor data is processed and used to generate different processed versions, and thus different video streams, which may highlight different aspects of the imaging sensor data.

In some cases, the imaging sensor data may be suitable for generating a reflectance image, and also suitable for generating a fluorescence overlay of fluorescence emissions in two different wavelength bands, e.g., as the imaging sensor data comprises intensity values of light recorded in a first wavelength band that is used to generate the fluorescence overlay in a first of the two different wavelength bands, intensity values of light recorded in a second wavelength band that is used to generate the fluorescence overlay in a second of the two different wavelength bands, and intensity values of light recorded in one or more further wavelength bands that are used to generate the reflectance image. **In** this case, the two or more video streams may comprise one or more of the following video streams: a first video stream video stream that is based on reflectance imaging, without a pseudo-color overlay, a second video stream that is based on reflectance imaging with a pseudo-color overlay that is based on the fluorescence emissions in the first of the two wavelength bands, a third video stream showing an isolated representation of the fluorescence emissions in the first of the two wavelength bands, a fourth video stream that is based on reflectance imaging with a pseudo-color overlay that is based on the fluorescence emissions in the second of the two wavelength bands, a fifth video stream showing an isolated representation of the fluorescence emissions in the second of the two wavelength bands, and a sixth video stream that is based on reflectance imaging with a pseudo-color overlay that is based on the fluorescence emissions in the first and the second of the two wavelength bands etc. In some cases, a subset of the afore-mentioned video streams may be generated.

In some cases, different video streams that are based on reflectance imaging may be generated as well. For example, the two or more video streams may comprise one of more of a video stream with a first lower image contrast, a video stream with a second higher image contrast, a video stream that is based on high-dynamic-range imaging, a video stream in which reflections have been reduced compared to an unprocessed version of the imaging sensor data, and a video stream that is based on multi-spectral imaging.

In various examples, another source of video streams is image data that is obtained from external devices, such as an endoscope, an image-guided surgery system or a neurological monitoring system, or computer-generated image data, such as a pre-operative plan or a visual guidance overlay. The surgeon may choose to display these types of data, as a video stream, in parallel to the imaging data of the microscope. For example, the system may be further configured to obtain image data from an external device. Alternatively or additionally, the system may be configured to obtain the image data by generating the image data from a data source. The system may be configured to generate the at least one of the two or more video streams further based on the image data from the external device and/or based on the image data generated from a data source. In other words, while some of the video streams are generated by processing the imaging sensor data, some of the video streams may be generated based on other sources, which may be external or internal to the surgical microscope system. Consequently, the two or more video streams may comprise at least one video stream that is generated based on the imaging sensor data and at least one video stream that is generated based on the image data from the external device and/or based on the image data generated from a data source.

These additional video streams are generated based on the image data. In some cases, the image data already comprises video data, which may be used to generate the respective video stream. For example, the image data may comprise at least one of image data of an endoscope, image data of an image-guided surgery system (a surgical navigation system), and image data of a neurological monitoring system. These types of image data may already comprise video data, for example, and be converted into the respective video streams. Additionally or alternatively, other surgeon-relevant data e.g., overlay text, or a preoperative plan of some visual kind may be used to generate a video stream. For example, the image data may comprise image data of a pre-operative plan and/or and image data for generating a visual overlay. These types of image data may be generated from a data source, such as an image file or numerical or textual data.

For example, the endoscope, the neurological monitoring system and the image-guided surgery system may be considered to be external to the surgical microscope system, even if some of the functionality of the systems is provided by the surgical microscope system e.g., the system.

At a given time, at least one of the two or more video streams is generated (simultaneously). Whether or not a given version of the two or more video streams may depend on whether the video stream is currently being shown as part of the display signal. For example, the at least one video stream may be shown as part of the representation shown in the presentation display mode. Furthermore, the at least one video stream may be shown as part of a preview of the resulting representation of the at least one of the two or more video streams. Furthermore, at least a subset of the two or more video streams may be shown if the preview included in the touch-activated control element is a live preview. If a video stream is required for one of the above purposes, it may be generated, if not, the generation of the video streams may be omitted.

The at least one video stream is provided as part of the display signal generated for the display of the touch screen. In general, the display signal may be a signal for driving (e.g., controlling) the display of the touch-screen 130. For example, the display signal may comprise video data and/or control instructions for driving the display. For example, the display signal may be provided via one of the one or more interfaces 112 of the system. Accordingly, the system 110 may comprise a video interface 112 that is suitable for providing the video signal to the display of the touch screen.

The display signal is provided to the display of the touch screen 130. In general, the display signal can be driven in one of two display modes - in the configuration display mode, in which a configuration screen is presented, the configuration screen being suitable for adjusting the representation of the at least one of the two or more video streams, and which may be used primarily before surgery, and in the presentation display mode, in which the representation of the at least one of the two or more video streams is shown during surgery. In other words, the configuration display mode, and in particular the visual configuration interface 140, may be suitable for controlling the representation of the at least one of the two or more video streams. In the presentation display mode, the representation of the at least one of the two or more video streams, as configured using the visual configuration interface, may be shown. In general, the first and the presentation display modes may be mutually exclusive. For example, either the configuration display mode or the presentation display mode may be active. Consequently, the display signal may either comprise the visual configuration interface, or the (full) representation of the at least one video stream.

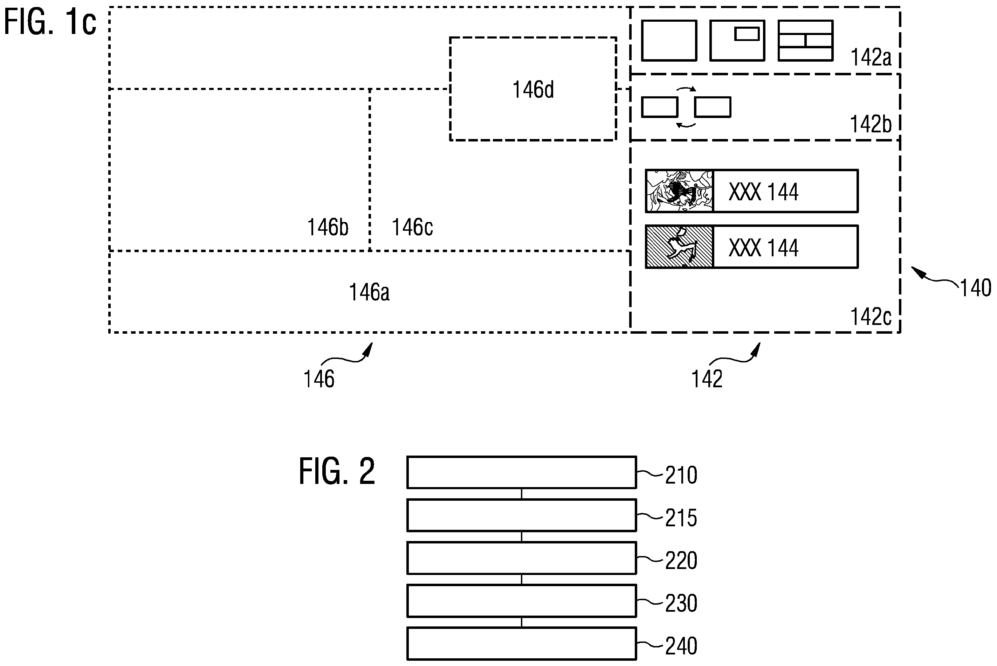

In the following, the configuration display mode, and thus the visual configuration interface, is introduced in more detail. Fig. 1c shows a schematic diagram of an example of a visual configuration interface. The visual configuration interface comprises a touch-activated control element 142 for selecting the at least one of the two or more video streams for display in the presentation display mode, with the touch-activated control element showing a preview 144 representing the two or more video streams. In Fig. 1c, the touch-activated control element is shown on the right, and is implemented as a "selection pane", which allows the selection of one or more of the video streams, which are shown as previews 144, for the representation shown in the presentation display mode. The touch-activated control element thus comprises a preview 144 for each of the two or more video streams, the preview comprising a description or name of the video stream (indicated as "XXX" in Fig. 1c) and a preview image (on the left of the description or name). Accordingly, the preview 144 may comprise a preview for each of the two or more video streams. For example, the preview, or each preview, may comprise a description or name of the video stream and a preview image of the video stream. In some examples, the preview representing the two or more video streams is provided as static images representing the respective video stream. For example, the static images may be generated once based on the video stream, e.g., based on the imaging sensor data, or may be static images that are unrelated to the video stream and/or imaging sensor data currently being recorded (e.g., factory-defined images). Alternatively, the preview representing the two or more video streams may be live images that are generated based on the video streams. In other words, the preview representing the two or more video streams may be provided based on the two or more video streams. In general, the previews of the video streams that are available for selection for the representation shown in the presentation display mode may be shown within the visual configuration interface. For example, if two or more video streams are generated for each fluorescence emission mode, the visual configuration interface for a fluorescence emission wavelength band (i.e. the visual configuration interface that is used to control the representation of the at least one of the two or more video streams that are generated based on fluorescence emissions in the wavelength band) may show a preview of the two or more video streams of the fluorescence emission wavelength bands. In addition to the video streams associated with the respective imaging mode or fluorescence emission wavelength band, the video streams that are generated based on the image data obtained from the external device and/or based on the computer-generated image data may be shown in the user interface. In other words, the visual configuration interface, e.g., the visual configuration interface for a fluorescence emission wavelength band, may show a preview of the video streams that are generated based on the image data obtained from the external device and/or based on the image data generated from a data source.

In addition to the control element showing the previews of the video streams, the touch-activated control element may comprise one or more additional control elements for controlling how the at least one of the two or more video streams is shown in the representation shown in the presentation display mode. For example, in Fig 1c, a touch-activated control element 142a is shown for selecting one of a "classic" full screen representation of a single video stream (on the left), a picture-in-picture representation (in the middle), and a picture-by-picture representation (on the right). Additionally or alternatively, a touch-activated control element 142b is shown for switching the placement of two video streams within the representation. The preview is shown in a preview pane 142c, that is part of the selection pane 142. On the left, a preview 146 of the representation shown in the presentation display mode is shown. In general, the preview 146 of the representation may correspond to the representation being shown in the presentation display mode, albeit at a smaller size to fit within the portion of the screen being used to show the preview 146. It supports multiple different representations, such as a full-screen representation (indicated by screen portion 146a), a picture-by-picture representation (indicated by screen portions 146b/146c), and a picture-in-picture representation, in which the full-screen representation (indicated by screen portion 146a) is combined with a smaller window (indicated by screen portion 146d). Accordingly, the visual configuration interface may comprise a preview 146 of the resulting representation of the at least one of the two or more video streams.

As indicated above, different representations may be shown in the presentation display mode, and thus also configured via the visual configuration interface. In a simple configuration, a single video stream may be selected and displayed in a full-screen mode/representation in the presentation display mode. In other words, one of the two or more video streams may be shown in a full-screen mode in the presentation display mode. Accordingly, the system may be configured to generate the visual configuration interface such that one of the two or more video streams is selectable via the touch-activated control element for a full-screen mode in the presentation display mode.

In a more advanced configuration, two of the two or more video streams may be shown at the same time in the representation shown in the presentation display mode. In other words, two of the two or more video streams may be displayed concurrently in the presentation display mode. Accordingly, the system may be configured to generate the visual configuration interface such that two of the two or more video streams are selectable via the touch-activated control element for concurrent display in the presentation display mode. For example, as shown in Fig. 1c, a picture-by-picture mode/representation may be used or a picture-in-picture mode/representation. In other words, two of the two or more video streams may be displayed concurrently via a picture-by-picture mode and/or picture-in-picture mode in the presentation display mode. Accordingly, the system may be configured to generate the visual configuration interface such that two of the two or more video streams are selectable via the touch-activated control element for concurrent display via a picture-by-picture mode and/or picture-in-picture mode in the presentation display mode. In a picture-by-picture mode/representation, the two video streams are shown side-by-side, with both video streams being shown with the (substantially) same size. In a picture-in-picture mode, one of the video streams is shown in a full-screen mode or large window, and the other video stream is shown superimposed over a portion of the full-screen or window (see screen portions 146a and 146d in Fig. 1c).

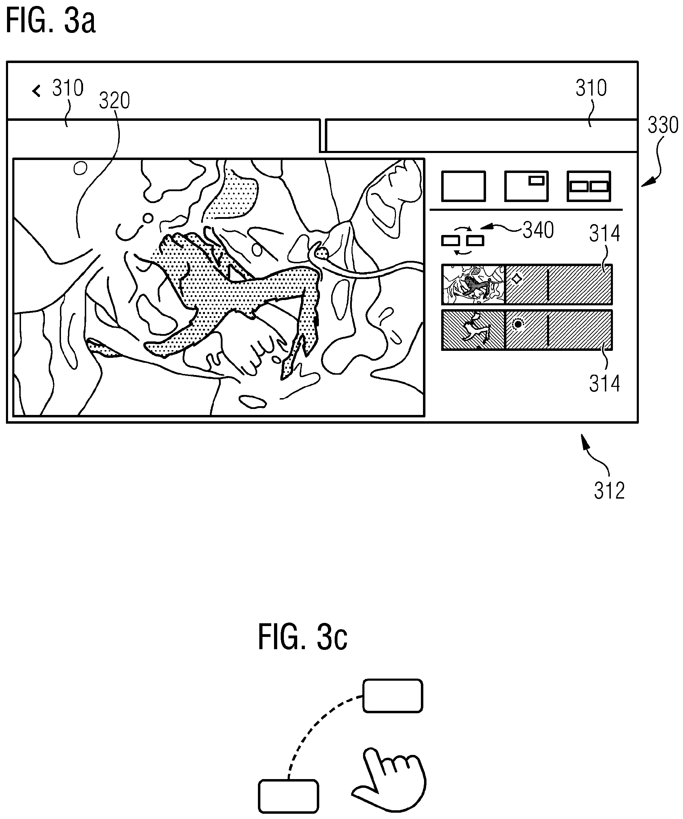

As mentioned above, the surgical microscope may support reflectance imaging and fluorescence imaging in one or more different wavelength bands. In some cases, a single visual configuration interface may be used to select the video streams of different imaging modes for the representation, e.g., to enable a representation containing video streams of different imaging modes. In some cases, however, the different imaging modes may be kept separate (apart from the reflectance image component of the combined reflectance/fluorescence emission video streams). For example, the system may be configured to generate a separate visual configuration interface for each of the one or more separate fluorescence emission bands. Additionally, the system may be configured to generate a separate visual configuration interface for video streams of the optical imaging data representing a reflectance image. An example for this is shown in Figs. 3a to 3h, where tabs 310 are used to select a visual configuration interface that is associated with one of the imaging modes. For example, the left tab may be used to select a first fluorescence imaging mode, and the right tab may be used to select a second fluorescence imaging mode. In addition to the video streams generated based on the imaging data of the respective imaging mode, the preview images representing the video stream or video streams generated based on the image data of the external device or based on the image data that is generated from data may be included in the visual configuration interface of the respective imaging modes.

The visual configuration interface is controlled via the touch input obtained via the touch interface. In turn, the touch input obtained via the touch interface is obtained with the sensor signal that is obtained from the touch interface of the touch screen 130 of the microscope system. The sensor signal represents the touch input obtained via the touch interface. For example, the sensor signal may comprise information on one or more coordinates at which a touch input has occurred.

The touch input is used to control the visual configuration interface. For example, the system may be configured to control the visual configuration interface via the touch input obtained via the touch interface. In general, the system may be configured to locate the touch input (e.g., in coordinates) relative to the visual configuration interface, e.g., relative to the buttons and/or element of the visual configuration interface, and to control the visual configuration interface based on a location of the touch input, and optionally based on a movement of the touch input.

In general, as pointed out above, the touch-activated control element comprises one or more control elements for configuring the representation shown in the presentation display mode. The one or more control elements are touch-controlled, i.e. they can be controlled via the touch input obtained via the touch interface. Accordingly, the touch-activated control element, and in particular the one or more control elements of the touch-activated control element, may have so-called touch targets, which hare regions of a coordinate system representing at least the visual configuration interface. If a touch input intersects with a touch target, the respective button (or control element) is actuated.

In some examples, the visual configuration interface may be controlled by single touches. For example, via the touch-activated control element 142a, a mode of representation (full-screen mode, picture-in-picture mode, or picture-by-picture mode) may be selected via a touch input, and a further touch input may be used to select one of the video streams to be placed in the respective mode of representation. Touch-activated control element 142b may be used to switch the placement of the video streams if two of the two or more video streams are concurrently shown in the picture-by-picture or picture-in-picture mode.

Alternatively or additionally, the visual configuration interface may be controlled using drag-and-drop operations. In other words, the system may be configured to generate the visual configuration interface such that the at least one of the two or more video streams is selectable via the touch-activated control element using a drag-and-drop operation. For example, the visual configuration interface may be generated such, that the preview representation of the two or more video streams are draggable to the portions of the preview screen, e.g., to the respective screen portions 146b/c of the picture-by-picture mode, to the screen portion 146a of the full-screen or picture-in-picture mode, and to the screen portion 146d of the smaller window of the picture-in-picture mode. For example, the video stream associated with the preview representation may be displayed in the screen portion where the preview representation is dropped in the drag-and-drop operation.

**In** various examples, the visual configuration interface may be generated with a visual indicator for illustrating the touch-based operations possible via the visual configuration interface. For example, the visual indicator may hint at the possibility of performing a drag-and-drop operation, or hint at possible drop targets when a preview representation has been picked up.

**In** some examples, the representation of the at least one of the two or more video streams may be saved as a setting, e.g., using the one or more storage devices, that can be triggered by the user of the surgical microscope system. For example, the visual configuration interface may comprise a touch-activated control element for saving the currently selected representation of the at least one of the two or more video streams. For example, the saved settings may be accessible via an input device of the surgical microscope system, e.g., via a button arranged at a handle of the microscope of the surgical microscope system. This would allow the surgeon to program such views and assign them to specific microscope handle buttons, such that the surgeon can toggle or switch between different pre-defined settings.

The one or more interfaces 112 may correspond to one or more inputs and/or outputs for receiving and/or transmitting information, which may be in digital (bit) values according to a specified code, within a module, between modules or between modules of different entities. For example, the one or more interfaces 112 may comprise interface circuitry configured to receive and/or transmit information. In embodiments the one or more processors 114 may be implemented using one or more processing units, one or more processing devices, any means for processing, such as a processor, a computer or a programmable hardware component being operable with accordingly adapted software. In other words, the described function of the one or more processors 114 may as well be implemented in software, which is then executed on one or more programmable hardware components. Such hardware components may comprise a general-purpose processor, a Digital Signal Processor (DSP), a micro-controller, etc. In at least some embodiments, the one or more storage devices 116 may comprise at least one element of the group of a computer readable storage medium, such as a magnetic or optical storage medium, e.g., a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

More details and aspects of the system and surgical microscope system are mentioned in connection with the proposed concept or one or more examples described above or below (e.g., Fig. 2 to 4). The system and surgical microscope system may comprise one or more additional optional features corresponding to one or more aspects of the proposed concept or one or more examples described above or below.

Fig. 2 shows a flow chart of an example of a method for a surgical microscope system, e.g., for the surgical microscope system of Figs. 1a to 1c. The method comprises obtaining 210 imaging sensor data from at least one optical imaging sensor of a microscope of the surgical microscope system. Optionally, the method comprises obtaining 215 image data from an external device or by generating the image data from a data source. The method comprises generating 220 at least one of two or more video streams based on the imaging sensor data. The method comprises obtaining 230 a sensor signal from a touch interface of a touch screen of the microscope system. The sensor signal represents a touch input obtained via the touch interface. The method comprises generating 240 a display signal for a display of the touch screen of the microscope system.. The display signal comprises, in a configuration display mode, a visual configuration interface, the visual configuration interface being controlled via the touch input obtained via the touch interface, and, in a presentation display mode, a representation of the at least one of the two or more video streams. The visual configuration interface comprises a touch-activated control element for selecting the at least one of the two or more video streams for display in the presentation display mode, the touch-activated control element showing a preview representing the two or more video streams.

As indicated above, features described in connection with the system 110, the microscope 120 and the surgical microscope system 100 of Figs. 1a to 1c may be likewise applied to the method of Fig. 2.

More details and aspects of the method are mentioned in connection with the proposed concept or one or more examples described above or below (e.g., Fig. 1a to 1c, 3a to 4). The method may comprise one or more additional optional features corresponding to one or more aspects of the proposed concept or one or more examples described above or below.

Various aspects of the present disclosure relate to the selection of a view, i.e. to view selections. The present disclosure generally relates to microscope on-screen controls. The User Interface/User eXperience (UI/UX) enables selection and control of microscope functions, in an intuitive and clear manner, while keeping a clean UI and where possible showing the immediate effect of the selected changes.

In some microscopes, the illumination control panel may consist of three sliders plus various other buttons, displayed on the side control panel of the microscope stand. These controls do not show the effect of a live image, are not visually pleasing, and would substantially hide any underlying image. There is no provision for adapting how white light and fluorescent images are displayed to the surgeon, particularly on a screen.

The proposed concept therefore provides a viewer configuration screen (which may correspond to the visual configuration interface introduced in connection with Figs. 1a to2). The viewer configuration screen may have two tabs (which may relate to two separate visual configuration interfaces), which are used to select between different fluorescence functions. Both white light with pseudo-color overlay, and monochrome fluorescence images may be available (as video streams that are based on processed versions of imaging sensor data) to the surgeon, and can be positioned side-by-side or picture-in-picture. The location of the picture-in-picture configuration can be selected by dragging and dropping, for example. Optionally the inset buttons may include live images.

Fig. 3a shows a schematic drawing of an example of a user interface for a microscope system according to the proposed concept. Fig. 3a shows a user interface with two tabs 310, which are used to select one of two visual configuration interfaces. A preview screen 320 shows how the representation of the view or views being selected looks like on the display of the microscope (and thus provides, for example, a representation of the at least one of the two or more video streams). In this context, the term "view" may be used for the term "video stream" used in connection with Figs. 1a to 2. A selection pane 312 on the right provides two touch-activated control elements 314 showing a preview representation of the different views, with a preview image and a description of the views. The selection pane 312 further comprises a touch-activated control element 330 for selecting a representation/mode, e.g., to select between a full-screen representation, a picture-in-picture representation, and a picture-by-picture representation. The selection pane 312 further comprises a touch-activated control element 340 for switching between two views, e.g., to switch the content of the two viewing windows in the picture-by-picture mode between the two windows, or to switch the content of the full-screen and the window in the picture-in-picture mode. The user interface may comprise one or more further element, such as a description of the viewer configuration screen, a "back" button for switching to the previous menu or to the live view, and/or additional information.

The configuration of the viewer lets the user combine pictures regarding layout and relationship. Thus the user can view two different images (e.g., two video streams based on fluorescence imaging of fluorescence emissions at wavelengths around 800 nm, with the first video stream showing a combined white-light and pseudo-color image, and the second video stream showing the fluorescence emissions in isolation) or (two video streams based on fluorescence imaging of fluorescence emissions at wavelengths around 400 nm ) in one view as Side by side (which allows switching the sides), Picture in picture (which allows switching between the smaller and the bigger image), or a "classic live view" (i.e. a single view in a full-screen representation).

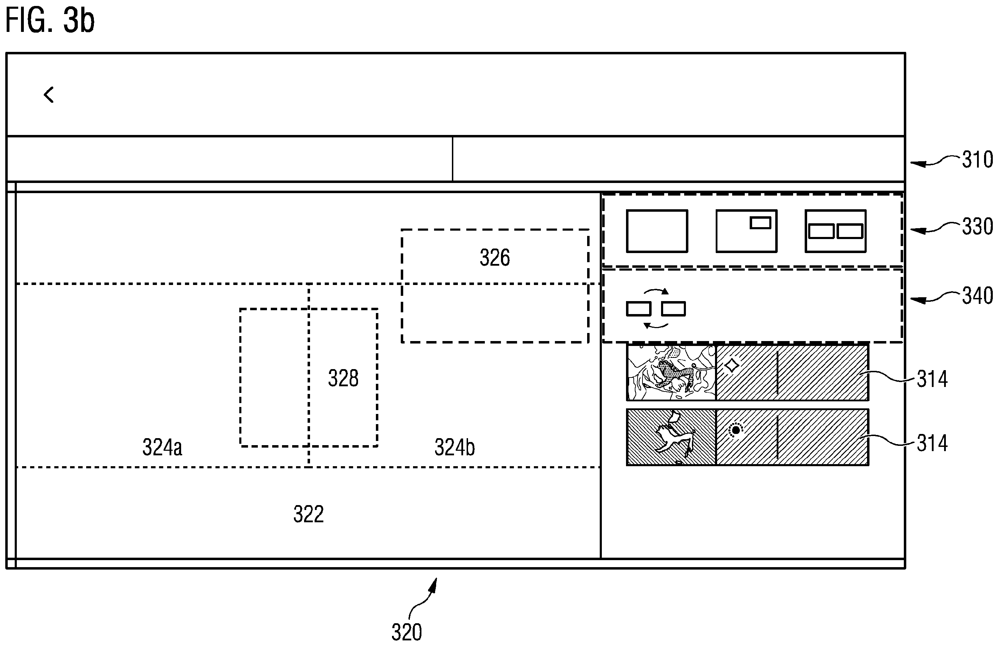

Fig. 3b shows a schematic drawing of another example of the user interface for the microscope system, in which an exemplary positioning of various elements is illustrated. Fig. 3b shows an exemplary placement of the portion 322 of the screen being used for the full-screen mode (also for the larger view in the picture-in-picture mode), the portions 324a/b of the screen being used for the picture-by-picture mode, and the portion 326 being used for the smaller view in the picture-in-picture mode). In general the portions of the screens being used, as well as the touch-activated control elements 314; 330; 340 of the selection pane may be center-aligned with another.

A long press on one of the representations of the views in the selection pane (or in one of the portions 322-326) may select an item for dragging. The long-press may be combined with a drag movement on the touch screen to move the object on the screen. Releasing the finger may drop the item on the desired location.

Portion 328 of the screen indicates an exemplary placement of a visual indicator. Also, Fig. 3c shows an example of a visual indicator of a user interface. The visual indicator is an affordance that illustrates the possibility of picking one of the views from the selection pane (or from one of the portions 322-326) and dragging the view to a desired portion 322-326 of the screen. The visual indicator is an element for guidance to drag and drop an element on the screen.

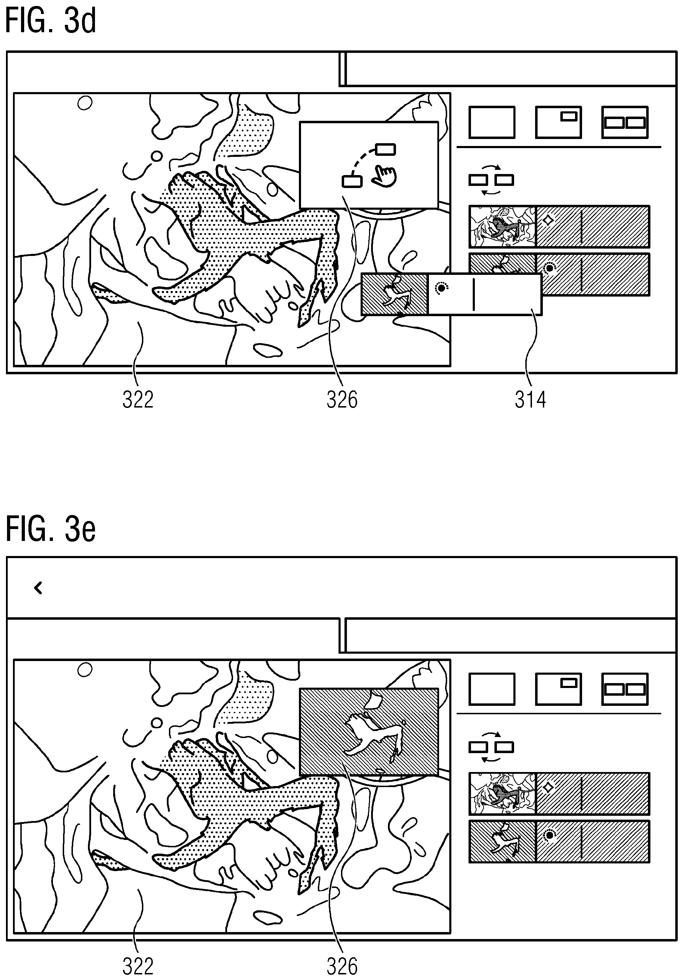

Figs. 3d and 3e shows schematic drawings of an example of a user interface for a microscope system, in which a picture-in-picture mode is shown. In Fig. 3d, the visual indicator is shown in portion 326 of the screen (where the selected view is to be dropped). The view, as represented by the touch-activated control element 314, is dragged from the selection pane to the portion 326 of the screen. After the dragging motion is complete and the view is released, the dragged view is shown in the portion 326 of the screen.

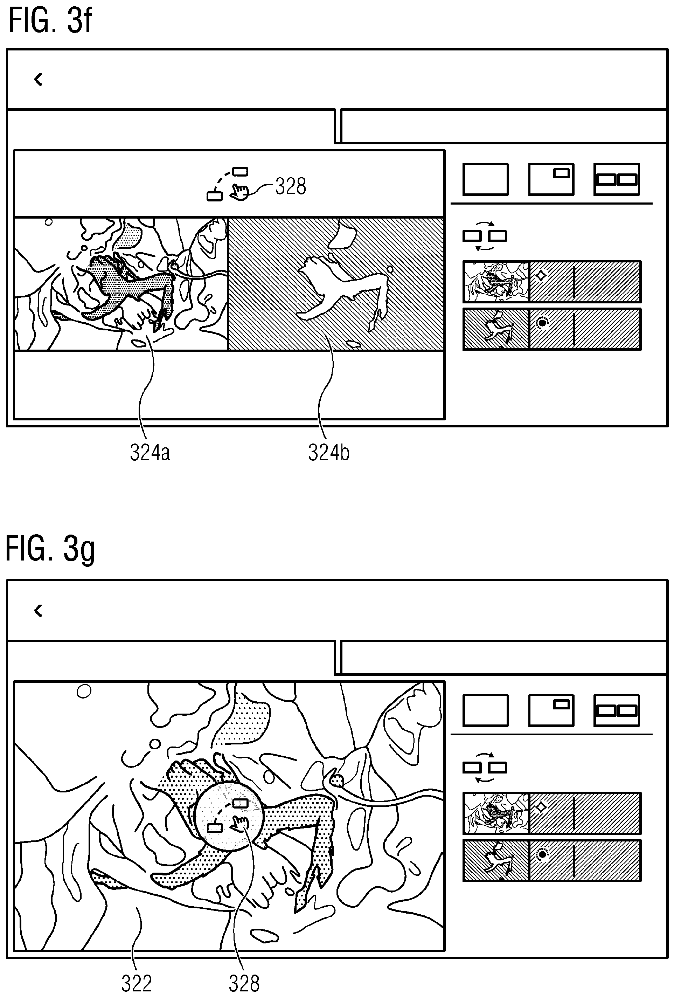

Figs. 3f shows a schematic drawing of an example of a user interface for a microscope system, in which a picture-by-picture mode is shown. In Fig. 3f, the two views are shown side-by-side in picture-by-picture mode in portions 324a/b of the screen. The visual indicator is shown in a portion 328 of the screen above the two portions 324a/b being used for the picture-by-picture representation.

Fig. 3g shows a schematic drawing of an example of a user interface for a microscope system, in which a placement of a visual indicator is shown in the portion 328 of the screen, in the center of the full-screen representation.

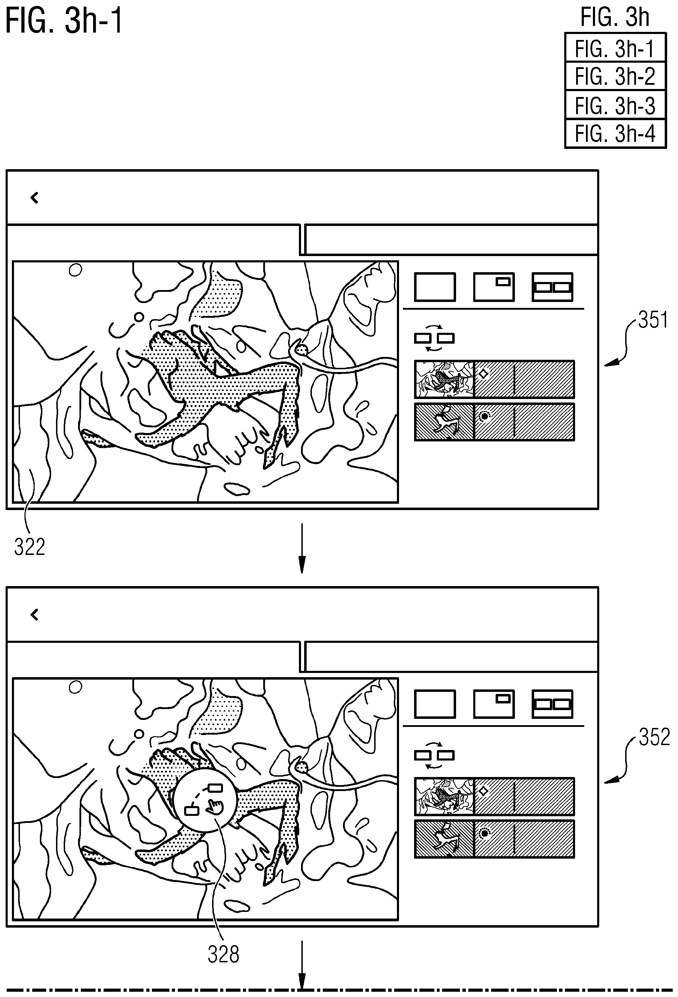

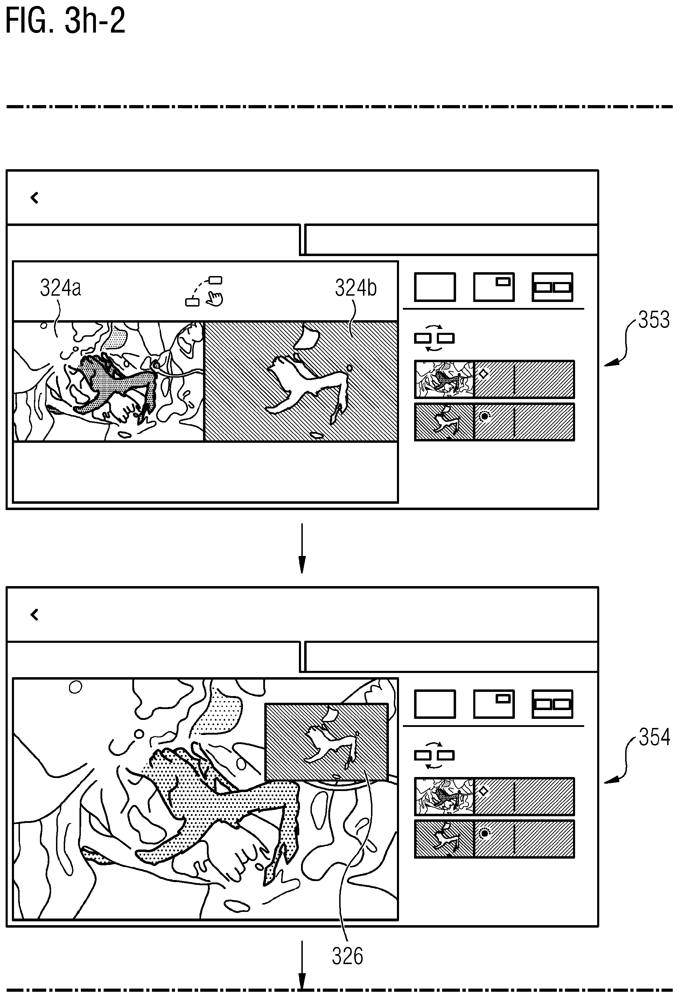

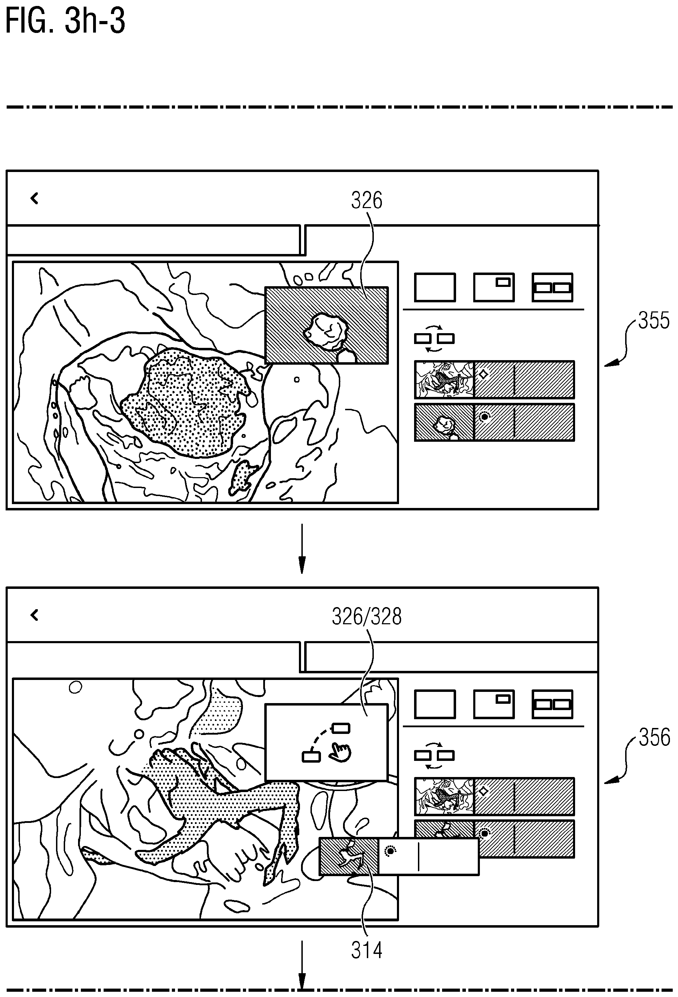

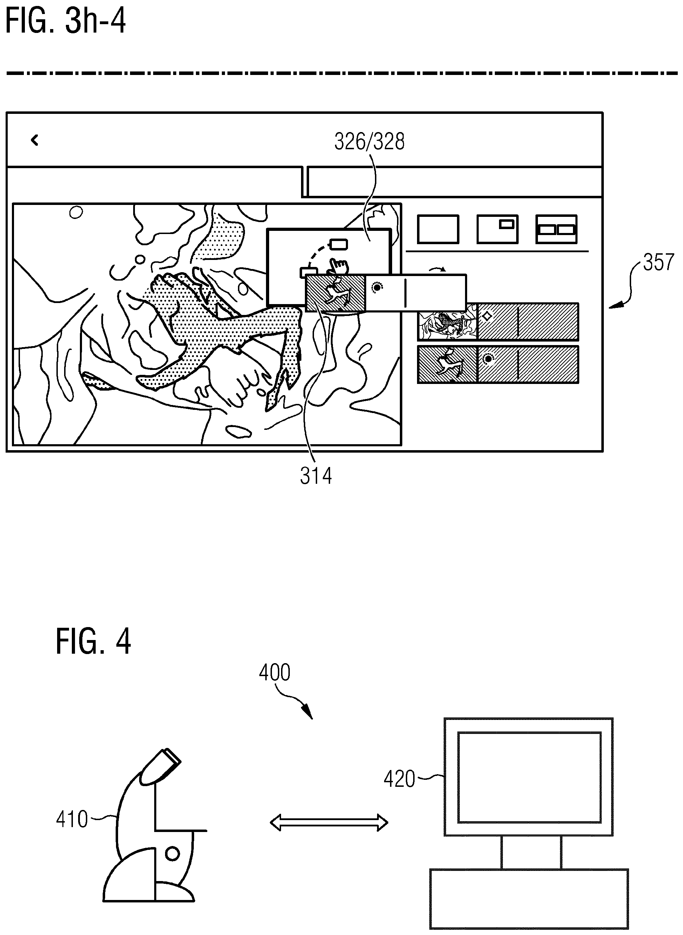

Fig. 3hshows a schematic drawing of an example of a transition between screens of an exemplary user interface according to several use-cases. Screen 351 shows the video stream showing a combined white-light and pseudo-color image in a full-screen configuration in the respective portion 322 of the screen. Screen 352 shows the visual indicator on top of the full-screen representation in portion 328 of the screen. Screen 353 shows a picture-by-picture view, with the views showing the combined white-light and pseudo-color image and showing the fluorescence emissions ins isolation being shown side-by-side in portions 324a/b of the screen, with the visual indicator being shown above the two portions of the screen. Screen 354 shows a picture-in-picture view, with the view showing the combined white-light and pseudo-color image being shown in the smaller window in portion 326 of the screen. In screen 355, the corresponding arrangement is shown for the fluorescence imaging mode using fluorescence emissions around 400 nm, with the view showing the combined white-light and pseudo-color image view being shown in full-screen and view showing the fluorescence emissions in isolation being shown in the portion 326 of the screen. In screen 356, the visual indicator is shown in portion 326 of the screen (where the selected view is to be dropped). The view, as represented by the touch-activated control element in the selection pane, is dragged from the selection pane to the portion 326 of the screen. After the dragging motion is complete and the view is released, the dragged view is shown in the portion 326 of the screen (screen 357).

More details and aspects of the microscope system and user interface are mentioned in connection with the proposed concept or one or more examples described above or below (e.g., Fig. 1a to 2, 4). The microscope system and user interface may comprise one or more additional optional features corresponding to one or more aspects of the proposed concept or one or more examples described above or below.

Some embodiments relate to a microscope comprising a system as described in connection with one or more of the Figs. 1 to 3h. Alternatively, a microscope may be part of or connected to a system as described in connection with one or more of the Figs. 1 to 3h. Fig. 4 shows a schematic illustration of a system 400 configured to perform a method described herein. The system 400 comprises a microscope 410 and a computer system 420. The microscope 410 is configured to take images and is connected to the computer system 420. The computer system 420 is configured to execute at least a part of a method described herein. The computer system 420 may be configured to execute a machine learning algorithm. The computer system 420 and microscope 410 may be separate entities but can also be integrated together in one common housing. The computer system 420 may be part of a central processing system of the microscope 410 and/or the computer system 420 may be part of a subcomponent of the microscope 410, such as a sensor, an actor, a camera or an illumination unit, etc. of the microscope 410.

The computer system 420 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 420 may comprise any circuit or combination of circuits. In one embodiment, the computer system 420 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 420 may be a custom circuit, an application-specific integrated circuit (ASlC), or the like, such as, for example, one or more circuits (such as a communication circuit) for use in wireless devices like mobile telephones, tablet computers, laptop computers, two-way radios, and similar electronic systems. The computer system 420 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 420 may also include a display device, one or more speakers, and a keyboard and/or controller, which can include a mouse, trackball, touch screen, voice-recognition device, or any other device that permits a system user to input information into and receive information from the computer system 420.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of reference Signs

- 100: Surgical microscope system
- 110: System
- 112: One or more interfaces
- 114: One or more processors
- 116: One or more storage devices
- 120: Microscope
- 130: Touch screen
- 140: Visual configuration interface
- 142: Touch-activated control element, selection pane
- 142a: Touch-activated control element for selecting a mode of representation
- 142b: Touch-activated control element for switching two video streams
- 142c: Portion of the selection pane comprising the previews
- 144: Preview
- 146: Preview
- 142a: Screen portion being used for full-screen mode and picture-in-picture mode
- 142b/c: Screen portions being used for picture-by-picture mode
- 142d: Screen portion being used for picture-in-picture mode
- 150: Arm
- 160: Handles
- 210: Obtaining imaging sensor data
- 215: Obtaining image data
- 220: Generating at least one of two or more video streams of the imaging sensor data
- 230: Obtaining a sensor signal from a touch interface
- 240: Generating a display signal
- 310: Tabs of a user interface
- 312: Selection pane
- 314: Touch-activated control elements with preview
- 320: Preview screen
- 322: Full-screen portion of the screen
- 324a/b: Portions of the screen used for picture-by-picture
- 326: Portion of the screen used for smaller window for picture-in-picture
- 328: Portion of the screen used for visual indicator
- 330: Touch-activated control element
- 340: Touch-activated control element
- 351-357: Screens
- 400: System
- 410: Microscope
- 420: Computer system

## Claims

1. A system (110) for a surgical microscope system (100), the system comprising one or more processors (114) and one or more storage devices (116), wherein the system is configured to:
obtain imaging sensor data from at least one optical imaging sensor of a microscope (120) of the surgical microscope system;
generate at least one of two or more video streams based on the imaging sensor data;
obtain a sensor signal from a touch interface of a touch screen (130) of the microscope system, the sensor signal representing a touch input obtained via the touch interface;
generate a display signal for a display of the touch screen of the microscope system, the display signal comprising, in a configuration display mode, a visual configuration interface (140), the visual configuration interface being controlled via the touch input obtained via the touch interface, and, in a presentation display mode, a representation of the at least one of the two or more video streams,
wherein the visual configuration interface comprises a touch-activated control element (142) for selecting the at least one of the two or more video streams for display in the presentation display mode, the touch-activated control element showing a preview (144) representing the two or more video streams, the two or more video streams comprising two or more different video streams that are based on different processed versions of the imaging sensor data, highlighting different aspects of the imaging sensor data.

2. The system according to claim 1, wherein the system is configured to generate the visual configuration interface such that two of the two or more video streams are selectable via the touch-activated control element for concurrent display in the presentation display mode.

3. The system according to one of the claims 1 or 2, wherein the system is configured to generate the visual configuration interface such that two of the two or more video streams are selectable via the touch-activated control element for concurrent display via a picture-by-picture mode and/or picture-in-picture mode in the presentation display mode.

4. The system according to one of the claims 1 to 3, wherein the system is configured to generate the visual configuration interface such that one of the two or more video streams is selectable via the touch-activated control element for a full-screen mode in the presentation display mode.

5. The system according to one of the claims 1 to 4, wherein the system is configured to generate the visual configuration interface such that the at least one of the two or more video streams is selectable via the touch-activated control element using a drag-and-drop operation.

6. The system according to one of the claims 1 to 5, wherein the system is further configured to obtain image data from an external device and/or by generating the image data from a data source, wherein the system is configured to generate the at least one of the two or more video streams further based on the image data from the external device and/or based on the image data generated from a data source.

7. The system according to claim 6, wherein the two or more video streams comprise at least one video stream that is generated based on the imaging sensor data and at least one video stream that is generated based on the image data from the external device and/or based on the image data generated from a data source.

8. The system according to one of the claims 6 or 7, wherein the image data comprises at least one of image data of an endoscope, image data of an image-guided surgery system, image data of a neurological monitoring system, image data of a pre-operative plan, and image data for generating a visual overlay.

9. The system according to one of the claims 1 to 8, wherein the surgical microscope system is suitable for performing fluorescence imaging in one or more separate fluorescence emission wavelength bands, wherein the system is configured to generate a separate visual configuration interface for each of the one or more separate fluorescence emission bands,
and/or wherein the surgical microscope system is suitable for performing reflectance imaging, wherein the system is configured to generate a separate visual configuration interface for video streams being based on the optical imaging data representing a reflectance image.

10. The system according to one of the claims 1 to 9, wherein the surgical microscope system is suitable for performing fluorescence imaging in one or more separate fluorescence emission wavelength bands, wherein the two or more video streams comprise, for each of the one or more separate fluorescence emission wavelength bands, two or more video streams being based on the imaging sensor data.

11. The system according to one of the claims 1 to 10, wherein the preview representing the two or more video streams is provided as static images representing the respective video stream,
or wherein the preview representing the two or more video streams is provided based on the two or more video streams.
and/or wherein the visual configuration interface comprises a preview (146) of the resulting representation of the at least one of the two or more video streams.

12. The system according to one of the claims 1 to 11, wherein the configuration display mode is suitable for controlling the representation of the at least one of the two or more video streams, and wherein, in the presentation display mode, the representation of the at least one of the two or more video streams, as configured using the visual configuration interface, is shown.

13. A surgical microscope system comprising the system according to one of the claims 1 to 12.

14. A method for a surgical microscope system, the method comprising:
obtaining (210) imaging sensor data from at least one optical imaging sensor of a microscope of the surgical microscope system;
generating (220) at least one of two or more video streams based on the imaging sensor data;
obtaining (230) a sensor signal from a touch interface of a touch screen of the microscope system, the sensor signal representing a touch input obtained via the touch interface; and
generating (240) a display signal for a display of the touch screen of the microscope system, the display signal comprising, in a configuration display mode, a visual configuration interface, the visual configuration interface being controlled via the touch input obtained via the touch interface, and, in a presentation display mode, a representation of the at least one of the two or more video streams,
wherein the visual configuration interface comprises a touch-activated control element for selecting the at least one of the two or more video streams for display in the presentation display mode, the touch-activated control element showing a preview representing the two or more video streams, the two or more video streams comprising two or more different video streams that are based on different processed versions of the imaging sensor data, highlighting different aspects of the imaging sensor data.

15. A computer program with a program code for performing the method according to claim 14 when the computer program is executed on a processor.

## Patentansprüche

1. System (110) für ein chirurgisches Mikroskopsystem (100), das System umfassend einen oder mehrere Prozessoren (114) und einen oder mehrere Speichervorrichtungen (116), wobei das System dazu ausgebildet ist:
Bildsensordaten von mindestens einem optischen Bildsensor eines Mikroskops (120) des chirurgischen Mikroskopsystems zu erhalten;
mindestens einen von zwei oder mehr Videostreams basierend auf den Bildsensordaten zu erzeugen;
ein Sensorsignal von einer Berührungsschnittstelle eines Touchscreens (130) des Mikroskopsystems zu erhalten, wobei das Sensorsignal eine über die Berührungsschnittstelle erhaltene Berührungseingabe darstellt;
ein Anzeigesignal für eine Anzeige des Touchscreens des Mikroskopsystems zu erzeugen, wobei das Anzeigesignal in einem Konfigurationsanzeigemodus eine visuelle Konfigurationsschnittstelle (140) umfasst, wobei die visuelle Konfigurationsschnittstelle über die über die Berührungsschnittstelle erhaltene Berührungseingabe gesteuert wird, und in einem Präsentationsanzeigemodus eine Darstellung des mindestens einen der zwei oder mehr Videostreams umfasst,
wobei die visuelle Konfigurationsschnittstelle ein berührungsaktivierbares Bedienelement (142) zum Auswählen des mindestens einen der zwei oder mehr Videostreams zur Anzeige im Präsentationsanzeigemodus umfasst, wobei das berührungsaktivierbare Bedienelement eine Vorschau (144) zeigt, die die zwei oder mehr Videostreams darstellt, wobei die zwei oder mehr Videostreams zwei oder mehr unterschiedliche Videostreams umfassen, die basierend auf unterschiedlichen verarbeiteten Versionen der Bildsensordaten unterschiedliche Aspekte der Bildsensordaten hervorheben.

2. System nach Anspruch 1, wobei das System dazu ausgebildet ist, die visuelle Konfigurationsschnittstelle derart zu erzeugen, dass zwei der zwei oder mehr Videostreams über das berührungsaktivierbare Bedienelement für die gleichzeitige Anzeige im Präsentationsanzeigemodus auswählbar sind.

3. System nach einem der Ansprüche 1 oder 2, wobei das System dazu ausgebildet ist, die visuelle Konfigurationsschnittstelle derart zu erzeugen, dass zwei der zwei oder mehr Videostreams über das berührungsaktivierbare Bedienelement für die gleichzeitige Anzeige über einen Bild-neben-Bild-Modus und/oder einen Bild-im-Bild-Modus im Präsentationsanzeigemodus auswählbar sind.

4. System nach einem der Ansprüche 1 bis 3, wobei das System dazu ausgebildet ist, die visuelle Konfigurationsschnittstelle derart zu erzeugen, dass einer der zwei oder mehr Videostreams über das berührungsaktivierbare Bedienelement für einen Vollbildmodus im Präsentationsanzeigemodus auswählbar ist.

5. System nach einem der Ansprüche 1 bis 4, wobei das System dazu ausgebildet ist, die visuelle Konfigurationsschnittstelle derart zu erzeugen, dass der mindestens eine der zwei oder mehr Videostreams über das berührungsaktivierbare Bedienelement unter Verwendung eines Drag-and-Drop-Vorgangs auswählbar ist.

6. System nach einem der Ansprüche 1 bis 5, wobei das System ferner dazu ausgebildet ist, Bilddaten von einer externen Vorrichtung und/oder durch Erzeugen der Bilddaten aus einer Datenquelle zu erhalten, wobei das System dazu ausgebildet ist, den mindestens einen der zwei oder mehr Videostreams ferner basierend auf den Bilddaten von der externen Vorrichtung und/oder basierend auf den aus einer Datenquelle erzeugten Bilddaten zu erzeugen.

7. System nach Anspruch 6, wobei die zwei oder mehr Videostreams mindestens einen Videostream umfassen, der basierend auf den Bildsensordaten erzeugt wird, und mindestens einen Videostream, der basierend auf den Bilddaten von der externen Vorrichtung und/oder basierend auf den aus einer Datenquelle erzeugten Bilddaten erzeugt wird.

8. System nach einem der Ansprüche 6 oder 7, wobei die Bilddaten mindestens eines aus Bilddaten eines Endoskops, Bilddaten eines bildgeführten Chirurgiesystems, Bilddaten eines Neuromonitoring-Systems, Bilddaten eines präoperativen Plans und Bilddaten zum Erzeugen einer visuellen Überlagerung umfassen.

9. System nach einem der Ansprüche 1 bis 8, wobei das chirurgische Mikroskopsystem geeignet ist, Fluoreszenzbildgebung in einem oder mehreren separaten Fluoreszenz-Emissionswellenlängenbändern durchzuführen, wobei das System dazu ausgebildet ist, für jedes der einen oder mehreren separaten Fluoreszenz-Emissionswellenlängenbänder eine separate visuelle Konfigurationsschnittstelle zu erzeugen,
und/oder wobei das chirurgische Mikroskopsystem geeignet ist, Reflexionsbildgebung durchzuführen, wobei das System dazu ausgebildet ist, eine separate visuelle Konfigurationsschnittstelle für Videostreams basierend auf den optischen Bilddaten, die ein Reflexionsbild darstellen, zu erzeugen.

10. System nach einem der Ansprüche 1 bis 9, wobei das chirurgische Mikroskopsystem geeignet ist, Fluoreszenzbildgebung in einem oder mehreren separaten Fluoreszenz-Emissionswellenlängenbändern durchzuführen, wobei die zwei oder mehr Videostreams für jedes der einen oder mehreren separaten Fluoreszenz-Emissionswellenlängenbänder zwei oder mehr Videostreams basierend auf den Bildsensordaten umfassen.

11. System nach einem der Ansprüche 1 bis 10, wobei die Vorschau, die die zwei oder mehr Videostreams darstellt, als statische Bilder, die den jeweiligen Videostream darstellen, bereitgestellt wird,
oder wobei die Vorschau, die die zwei oder mehr Videostreams darstellt, basierend auf den zwei oder mehr Videostreams bereitgestellt wird.
und/oder wobei die visuelle Konfigurationsschnittstelle eine Vorschau (146) der resultierenden Darstellung des mindestens einen der zwei oder mehr Videostreams umfasst.

12. System nach einem der Ansprüche 1 bis 11, wobei der Konfigurationsanzeigemodus geeignet ist, die Darstellung des mindestens einen der zwei oder mehr Videostreams zu steuern, und wobei im Präsentationsanzeigemodus die Darstellung des mindestens einen der zwei oder mehr Videostreams, wie unter Verwendung der visuellen Konfigurationsschnittstelle konfiguriert, gezeigt wird.

13. Chirurgisches Mikroskopsystem, umfassend das System nach einem der Ansprüche 1 bis 12.

14. Verfahren für ein chirurgisches Mikroskopsystem, das Verfahren umfassend:
Erhalten (210) von Bildsensordaten von mindestens einem optischen Bildsensor eines Mikroskops des chirurgischen Mikroskopsystems;
Erzeugen (220) mindestens eines von zwei oder mehr Videostreams basierend auf den Bildsensordaten;
Erhalten (230) eines Sensorsignals von einer Berührungsschnittstelle eines Touchscreens des Mikroskopsystems, wobei das Sensorsignal eine über die Berührungsschnittstelle erhaltene Berührungseingabe darstellt; und
Erzeugen (240) eines Anzeigesignals für eine Anzeige des Touchscreens des Mikroskopsystems, wobei das Anzeigesignal in einem Konfigurationsanzeigemodus eine visuelle Konfigurationsschnittstelle umfasst, wobei die visuelle Konfigurationsschnittstelle über die über die Berührungsschnittstelle erhaltene Berührungseingabe gesteuert wird, und in einem Präsentationsanzeigemodus eine Darstellung des mindestens einen der zwei oder mehr Videostreams umfasst,
wobei die visuelle Konfigurationsschnittstelle ein berührungsaktivierbares Bedienelement zum Auswählen des mindestens einen der zwei oder mehr Videostreams zur Anzeige im Präsentationsanzeigemodus umfasst, wobei das berührungsaktivierbare Bedienelement eine Vorschau zeigt, die die zwei oder mehr Videostreams darstellt, wobei die zwei oder mehr Videostreams zwei oder mehr unterschiedliche Videostreams umfassen, die basierend auf unterschiedlichen verarbeiteten Versionen der Bildsensordaten unterschiedliche Aspekte der Bildsensordaten hervorheben.

15. Computerprogramm mit einem Programmcode zur Durchführung des Verfahrens nach Anspruch 14, wenn das Computerprogramm auf einem Prozessor ausgeführt wird.

## Revendications

1. Système (110) pour un système de microscope chirurgical (100), le système comprenant un ou plusieurs processeurs (114) et un ou plusieurs dispositifs de stockage (116), dans lequel le système est configuré pour :
obtenir des données de capteur d'imagerie à partir d'au moins un capteur d'imagerie optique d'un microscope (120) du système de microscope chirurgical ;
générer au moins un de deux ou plusieurs flux vidéo sur la base des données de capteur d'imagerie ;
obtenir un signal de capteur à partir d'une interface tactile d'un écran tactile (130) du système de microscope, le signal de capteur représentant une entrée tactile obtenue via l'interface tactile ;
générer un signal d'affichage pour l'affichage de l'écran tactile du système de microscope, le signal d'affichage comprenant, dans un mode d'affichage de configuration, une interface de configuration visuelle (140), l'interface de configuration visuelle étant commandée via l'entrée tactile obtenue via l'interface tactile, et, dans un mode d'affichage de présentation, une représentation d'au moins un des deux ou plusieurs flux vidéo,
dans lequel l'interface de configuration visuelle comprend un élément de commande à activation tactile (142) pour sélectionner au moins un des deux ou plusieurs flux vidéo pour l'affichage dans le mode d'affichage de présentation, l'élément de commande à activation tactile montrant une prévisualisation (144) représentant les deux ou plusieurs flux vidéo, les deux ou plusieurs flux vidéo comprenant deux ou plusieurs flux vidéo différents qui sont basés sur différentes versions traitées des données de capteur d'imagerie, mettant en évidence différents aspects des données de capteur d'imagerie.

2. Système selon la revendication 1, dans lequel le système est configuré pour générer l'interface de configuration visuelle de telle sorte que deux des deux ou plusieurs flux vidéo sont sélectionnables via l'élément de commande à activation tactile pour un affichage simultané dans le mode d'affichage de présentation.

3. Système selon l'une des revendications 1 ou 2, dans lequel le système est configuré pour générer l'interface de configuration visuelle de telle sorte que deux des deux ou plusieurs flux vidéo sont sélectionnables via l'élément de commande à activation tactile pour un affichage simultané via un mode image par image et/ou un mode image dans l'image dans le mode d'affichage de présentation.

4. Système selon l'une des revendications 1 à 3, dans lequel le système est configuré pour générer l'interface de configuration visuelle de telle sorte qu'un des deux ou plusieurs flux vidéo est sélectionnable via l'élément de commande à activation tactile pour un mode plein écran dans le mode d'affichage de présentation.

5. Système selon l'une des revendications 1 à 4, dans lequel le système est configuré pour générer l'interface de configuration visuelle de telle sorte qu'au moins un des deux ou plusieurs flux vidéo est sélectionnable via l'élément de commande à activation tactile au moyen d'une opération de glisser-déposer.

6. Système selon l'une des revendications 1 à 5, dans lequel le système est en outre configuré pour obtenir des données d'image à partir d'un dispositif externe et/ou par génération des données d'image à partir d'une source de données, dans lequel le système est configuré pour générer en outre au moins un des deux ou plusieurs flux vidéo sur la base des données d'image provenant du dispositif externe et/ou sur la base des données d'image générées à partir d'une source de données.

7. Système selon la revendication 6, dans lequel les deux ou plusieurs flux vidéo comprennent au moins un flux vidéo qui est généré sur la base des données de capteur d'imagerie et au moins un flux vidéo qui est généré sur la base des données d'image provenant du dispositif externe et/ou sur la base des données d'image générées à partir d'une source de données.

8. Système selon l'une des revendications 6 ou 7, dans lequel les données d'image comprennent au moins un parmi des données d'image d'un endoscope, des données d'image d'un système de chirurgie guidée par l'image, des données d'image d'un système de surveillance neurologique, des données d'image d'un plan préopératoire, et des données d'image pour générer une superposition visuelle.

9. Système selon l'une des revendications 1 à 8, dans lequel le système de microscope chirurgical est apte à réaliser une imagerie par fluorescence dans une ou plusieurs bandes de longueurs d'onde d'émission de fluorescence séparées, dans lequel le système est configuré pour générer une interface de configuration visuelle séparée pour chacune de la ou des bandes d'émission de fluorescence séparées,
et/ou dans lequel le système de microscope chirurgical est apte à réaliser une imagerie en réflectance, dans lequel le système est configuré pour générer une interface de configuration visuelle séparée pour des flux vidéo, ces flux étant basés sur les données d'imagerie optique représentant une image de réflectance.

10. Système selon l'une des revendications 1 à 9, dans lequel le système de microscope chirurgical est apte à réaliser une imagerie par fluorescence dans une ou plusieurs bandes de longueurs d'onde d'émission de fluorescence séparées, dans lequel les deux ou plusieurs flux vidéo comprennent, pour chacune de la ou des bandes de longueurs d'onde d'émission de fluorescence séparées, deux ou plusieurs flux vidéo, ces flux étant basés sur les données de capteur d'imagerie.

11. Système selon l'une des revendications 1 à 10, dans lequel la prévisualisation représentant les deux ou plusieurs flux vidéo est fournie sous forme d'images statiques représentant le flux vidéo respectif,
ou dans lequel la prévisualisation représentant les deux ou plusieurs flux vidéo est fournie sur la base des deux ou plusieurs flux vidéo.
et/ou dans lequel l'interface de configuration visuelle comprend une prévisualisation (146) de la représentation résultante d'au moins un des deux ou plusieurs flux vidéo.

12. Système selon l'une des revendications 1 à 11, dans lequel le mode d'affichage de configuration est apte à commander la représentation d'au moins un des deux ou plusieurs flux vidéo, et dans lequel, dans le mode d'affichage de présentation, la représentation d'au moins un des deux ou plusieurs flux vidéo, telle que configurée au moyen de l'interface de configuration visuelle, est affichée.

13. Système de microscope chirurgical comprenant le système selon l'une des revendications 1 à 12.

14. Méthode pour un système de microscope chirurgical, la méthode comprenant :
obtenir (210) des données de capteur d'imagerie à partir d'au moins un capteur d'imagerie optique d'un microscope du système de microscope chirurgical ;
générer (220) au moins un de deux ou plusieurs flux vidéo sur la base des données de capteur d'imagerie ;
obtenir (230) un signal de capteur à partir d'une interface tactile d'un écran tactile du système de microscope, le signal de capteur représentant une entrée tactile obtenue via l'interface tactile ; et
générer (240) un signal d'affichage pour l'affichage de l'écran tactile du système de microscope, le signal d'affichage comprenant, dans un mode d'affichage de configuration, une interface de configuration visuelle, l'interface de configuration visuelle étant commandée via l'entrée tactile obtenue via l'interface tactile, et, dans un mode d'affichage de présentation, une représentation d'au moins un des deux ou plusieurs flux vidéo,
dans lequel l'interface de configuration visuelle comprend un élément de commande à activation tactile pour sélectionner au moins un des deux ou plusieurs flux vidéo pour l'affichage dans le mode d'affichage de présentation, l'élément de commande à activation tactile montrant une prévisualisation représentant les deux ou plusieurs flux vidéo, les deux ou plusieurs flux vidéo comprenant deux ou plusieurs flux vidéo différents qui sont basés sur différentes versions traitées des données de capteur d'imagerie, mettant en évidence différents aspects des données de capteur d'imagerie.

15. Programme d'ordinateur avec un code de programme permettant de réaliser la méthode selon la revendication 14 lorsque le programme d'ordinateur est exécuté sur un processeur.
